# EUROPEAN PATENT APPLICATION

(11) **EP 1 616 541 A2**
(43) Date of publication of application: **18.01.2006**
(21) Application number: 05254351.9
(22) Date of filing: 12.07.2005
(51) Int. Cl.: A61F 5/00

(54) **Gripping aid**

(30) Priority: 12.07.2004 GB 0415538
(71) Applicant: County Durham and Darlington Priority Services NHS Trust, Durham DH1 5RD (GB)
(72) Inventor: Bell, Louis County Durham and Darlington Priority, Lanchester Road Durham DH1 5RD (GB); Platt, Tim, Middlesbrough TS1 3BA (GB)
(74) Representative: Elsworth, Dominic Stephen

(57) **Abstract**

A hand splint comprises a conically shaped grip portion having a first end and a second end, wherein the first and second ends each include a tool/implement holder in each of which a tool /implement may be removably inserted and held.

## Description

### Field of the Invention

This invention relates to hand splints, and in particular to hand splints which allow disabled persons to use tools and implements.

### Background of the Invention

Decreased functionality in the hand, sometimes referred to as "Tight Hand", affects people of all ages and medical backgrounds, for example those suffering from rheumatoid arthritis, stroke, cerebral palsy, spasticity, etc.

Current treatment for this condition is a programme of physiotherapy focusing on lengthening the tissues in the hand maintained by the use of a cone-shaped splint.

A conc-shapcd splint not only maintains the length of the tissues, but also ensures that the hand is in a functional position. One type of cone shaped splint is described in US 5,152,740 where the splint is in the form of an inflatable bladder. Another inflatable hand splint is described in US 5,020,515.

Another type of hand splint is known from US 4,960,114, which describes a hand splint comprising a shaped and oriented grip member and a brace for attachment to the fore-arm.

Devices in which utensils can be inserted are also known. One such device consists of a ball of high density foam in which an implement can be inserted or attached by means of an adaptor. The device is marketed under the name Dexball ™. It is also known to provide foam handles for cutlery, such handles being marketed under the name Selectalite Cutlery ™.

One problem with the known splints is that whilst they serve to maintain the length of the tissues in the hand, when a patient is holding the splint, the whole arm of the hand holding the splint tends to be immobile, unless the patient is working through an exercise with or on the instruction of a physiothcrapist.

Another problem with the known splints is that unless instructed, some patients hold the splint with the cone the wrong way around in the hand.

None of the known hand splints provide for a hand to be maintained in a functional position, whilst at the same time using the arm and hand to do work. By facilitating patients to undertake everyday tasks, the ability to use the hand and arm can be increased, and therefore the patient's independence and quality of life is improved.

It would therefore be desirable to improve hand splints.

### Summary of the Invention

According to one aspect of the invention, there is provided a hand splint as specified in Claim 1.

### Brief Description of the Drawings

In the drawings, which illustrate a preferred embodiment of the invention and are for the purpose of example:
Figure 1 is a schematic representation of hand splint according to the invention showing a first end of the splint;
figure 2 is a schematic representation of the hand splint illustrated in Figure 1 showing a second end of the splint;
Figure 3 is a schematic representation of the hand splint illustrated in Figure 1 with a tool/implement attached thereto; and
Figure 4 is a schematic representation of the hand splint illustrated in Figure 2 with a tool/implement attached thereto.

### Detailed Description of the Preferred Embodiments

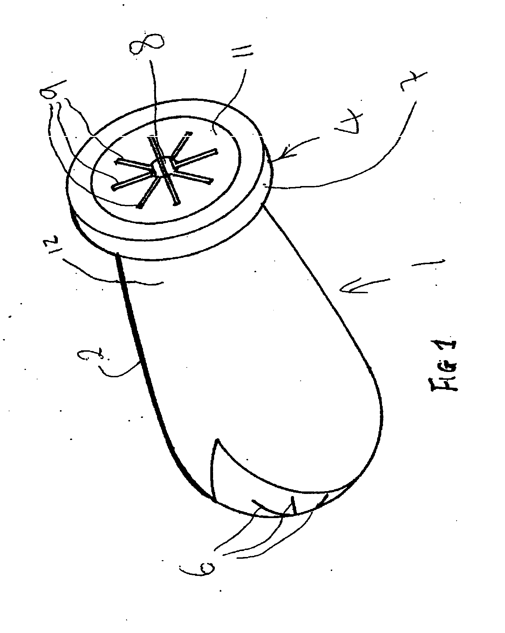

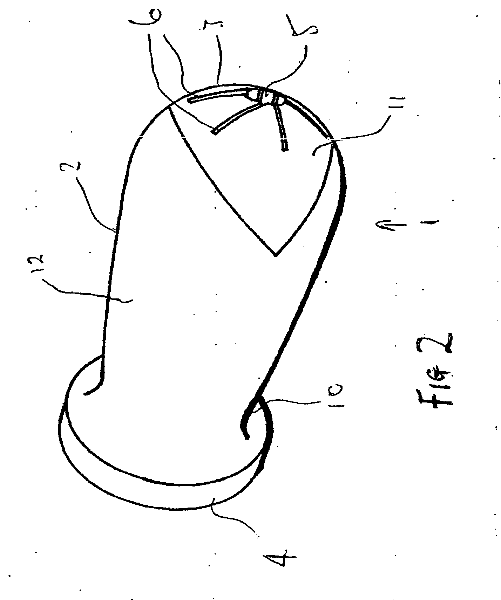

Referring now to Figures 1 and 2, there is shown a hand splint 1 comprising a grip portion 2, a first end 4 and a second end 3. In cross-section, along an axis extending between the first end 4 and the second end 3, the grip portion 2 is generally conical in shape with edges thereof being provided with curved surfaces.

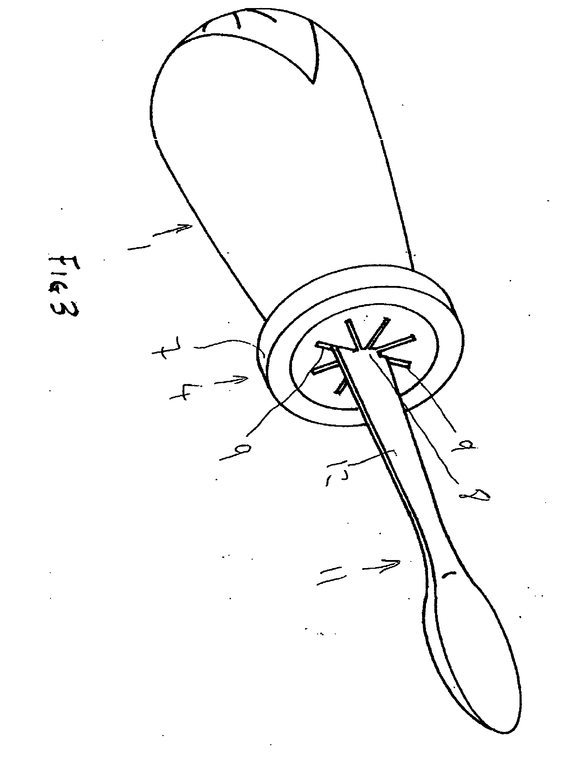

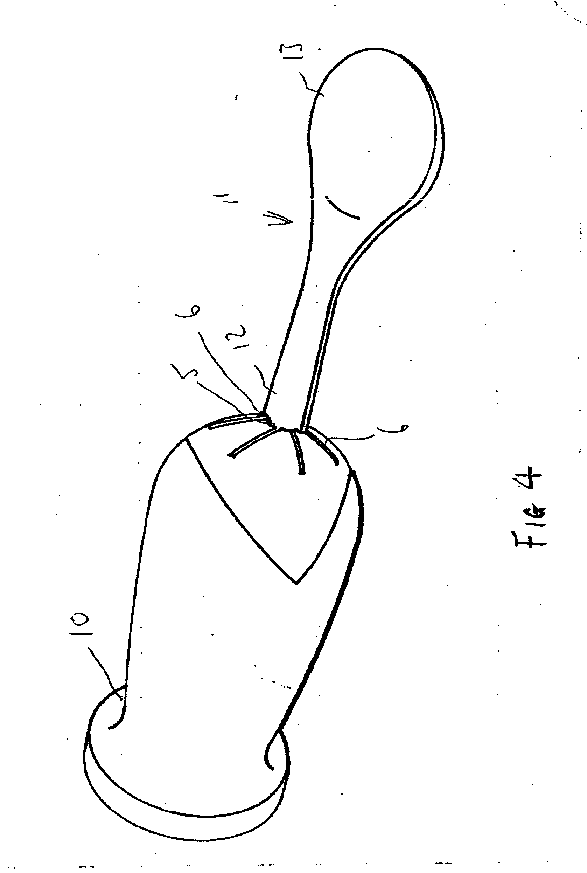

The first end 4 of the hand splint 1 includes a bore 5 and a plurality of slots 6 extending therefrom. The use of these slots will be described in greater detail with reference to Figure 3 and 4.

The second end 3 is in the form of a collar 7 of larger diameter than a neck 10 of the grip 2 at the point where the two meet.

In use the hand splint 1 is grasped with the thumb and index finger sitting in the neck 10. The collar 7 is used as a point of reference to assist in the correct location of the splint 1 in the hand, and to prevent the splint from slipping through the hand.

As illustrated in Figure 1, the second end 4 includes a bore 8 and a plurality of slots 9 extending therefrom. The collar 7 forms the outer edge of the second end.

Figures 3 and 4 illustrate the splint 1 with a tool/implement in the form of a spoon 11 attached thereto. The spoon shown in Figure 4 includes a handle 12 which is inserted into the bore 5 and slots 6. With the splint 1 gripped in the hand, the radial angle of the spoon 11 with respect to the longitudinal axis of the splint, and therefore of the hand is fixed. The radial angle of the spoon 11 with respect to the longitudinal axis of the splint may be changed by moving the spoon 11 from one set of opposing slots, and to another pair of opposing slots. The spoon shown in Figure 3 is located in the second end 3 of the splint 1, the spoon handle 12 being inserted into the bore 8 and opposing slots 9.

In the illustrated example, the splint 1 consists of a soft inner core 11 surrounded by a hard outer shell 12. The splint 1 is made of polyurethane, with the hard outer shell 12 being formed by the moulding process. Alternatively, the splint could be formed from a soft inner core of a first material, such as polyurethane and a separate hard outer shell of a mouldable plastics material.

In use, the splint is always held with the thumb and index finger sitting in the neck 10. By inserting tools or implements in the splint at different angles (by selecting which pair of opposing slots the tool is located in), and by inserting tools or implements into different ends of the splint, exercises aimed developing different arm movements, muscles and tissues.

In Figure 3 and 4, a spoon is inserted into the splint to facilitate self-feeding. Self-feeding as an exercise develops extension at the elbow and the wrist. Furthermore self-feeding is a requirement for independence.

In general, patients with spasticity hold their arms to their chests. In order for such patients to be able to feed themselves, they must be able to move their arms away from their chests. By inserting a tool or implement into the first end of the hand splint 1, this movement can be encouraged, because to use the tool or implement the arm must be moved away from the chest. A suitable exercise in this situation would involve inserting a crayon into the first end 4 of the hand splint 1. The patient must then extend the arm away from the chest in order to draw.

A significant advantage of the present invention is that its use does not immobilize the arm. In fact, by using tools or implements in conjunction with the splint, patients are encouraged to use the tools or implements, i.e. undertake functional activity, thereby speeding the development of arm movements, muscles and tissues.

## Claims

1. A hand splint comprising a conically shaped grip portion having a first end and a second end, wherein the first and second ends each include a tool/implement holder in each of which a tool/implement may be removably inserted and held.

2. A hand splint according to Claim 1, wherein the first end is in the form of a collar extending radially and axially of the grip portion.

3. A hand splint according to Claim 1 or 2, wherein the second end is rounded.

4. A hand splint according to Claim 3, wherein the second end is substantially hemispherical.

5. A hand splint according to any preceding claim, wherein the tool/implement holder provides for a tool to be inserted and held in the splint at a plurality of orientations relative to the grip portion.

6. A hand splint according to Claim 5, wherein the tool/implement holder provides for the tool/implement to be inserted and held in the splint at a plurality of spaced apart orientations, each orientation lying on a different radial axis of the splint.

7. A hand splint according to Claim 5 or 6, wherein the tool/implement holder provides a plurality of slots.

8. A hand splint according to Claim 7, wherein the slots bisect each other and the point of bisection lies substantially on a central longitudinal axis of the splint.

9. A hand splint according to any preceding claim, wherein the tool holder includes a bore.

10. A hand splint according to Claim 9, wherein the bore extends into the splint along a substantially central longitudinal axis of said splint.

11. A splint according to Claim 9 or 10 when dependent on Claim 7, wherein the slots are arranged in pairs, each slot of a pair extending to an opposite side of the bore and the slots of a pair being aligned with each other.

12. A hand splint according to any preceding claim, wherein the splint comprises a soft inner core and a hard outer shell.

13. A hand splint according to Claim 12, wherein the soft inner core and hard outer shell are both formed from polyurethane.

14. A hand splint according to Claim 13, wherein the soft inner core and the hard outer shell arc formed in the same moulding process.

15. A hand splint according to Claim 12 or 13, wherein the soft inner core and hard outer shell are formed from separately, the hard outer shell being attached to the soft inner core.

16. A hand splint substantially as shown in, and as described with reference to, the drawings.
